(19) Europäisches Patentamt
European Patent Office
Office européen des brevets

(11) Veröffentlichungsnummer: **0 115 811 B1**

(12) # EUROPÄISCHE PATENTSCHRIFT

(45) Veröffentlichungstag der Patentschrift: **18.04.90**

(51) Int. Cl.$^5$: **C 07 D 277/32** // C07D277/56

(21) Anmeldenummer: **84100695.0**

(22) Anmeldetag: **24.01.84**

(54) **2,4-Dichlor-5-thiazolcarboxaldehyd und ein Verfahren zu seiner Herstellung.**

(30) Priorität: **04.02.83 DE 3303704**

(43) Veröffentlichungstag der Anmeldung:
**15.08.84 Patentblatt 84/33**

(45) Bekanntmachung des Hinweises auf die Patenterteilung:
**18.04.90 Patentblatt 90/16**

(84) Benannte Vertragsstaaten:
**BE CH DE FR GB LI**

(56) Entgegenhaltungen:
**EP-A-0 053 765**
**FR-A-2 177 077**

(73) Patentinhaber: **BAYER AG**
**D-5090 Leverkusen 1 Bayerwerk (DE)**

(72) Erfinder: **Beck, Gunther, Dr.**
**Am Mittelberg 19**
**D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 115 811 B1

**Beschreibung**

Die vorliegende Anmeldung betrifft den neuen 2,4-Dichlor-5-thiazolcarboxaldehyd sowie ein Verfahren zu seiner Herstellung.

In Khim. Geterotsikl. Soedin *1975*, Seite 85 (englische Übersetzung: Chem. Het. Comp. *1975*, Seite 73) wird die Reaktion zwischen 2,4-Thiazolidindion (III), Dimethylformamid und Phosphoroxychlorid beschrieben. Hierbei werden die drei genannten Stoffe in einem optimalen Molverhältnis von 1:1,5:3 16 Stunden auf 80°C und kurze Zeit auf 115 bis 130°C erhitzt und anschließend hydrolysiert, wobei 4-Chlor-2,3-dihydro-2-oxo-5-thiazolcarboxaldehyd der Formel (I)

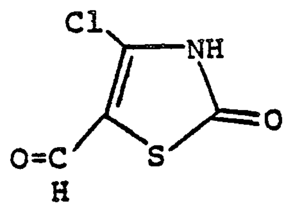

(I)

mit 40 bis 60%iger Ausbeute erhalten wird.

Es wurde nun überraschend gefunden, daß man den neuen 2,4-Dichlor-5-thiazolcarboxaldehyd der Formel (II)

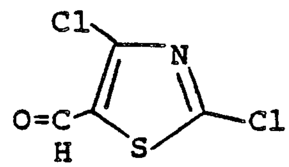

(II)

in guten Ausbeuten erhält, wenn man 2,4-Thiazolidindion der Formel (III)

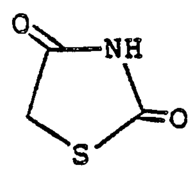

(III)

mit 1 bis 1,5 Mol Dimethylformamid und 3 bis 10 Mol Phosphoroxychlorid bei der Rückflußtemperatur des Reaktionsgemisches (etwa 115°C) bis zum Ende der Chlorwasserstoffgas-Entwicklung umsetzt und anschließend hydrolytisch aufarbeitet.

Der Reaktionsablauf kann durch das folgende Formelschema wiedergegeben werden:

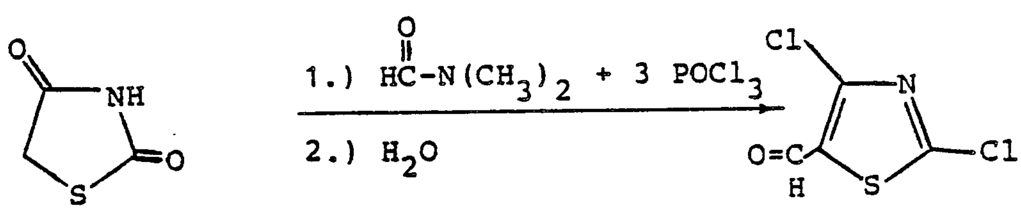


(III) (II)

Das als Ausgangsstoff zu verwendende 2,4-Thiazolidindion (III) ist bekannt und kann z.B. durch Umsetzung von Chloressigsäure mit Thioharnstoff in wäßrigem Medium hergestellt werden (vgl. z.B. Journal für Praktische Chemie [2] *9*, S. 9 (1874)).

Bei der Duchführung des erfindungsgemäßen Verfahrens setzt man auf 1 Mol 2,4-Thiazolidindion (III) im allgemeinen 1 bis 1,5 Mol, vorzugsweise 4,5 bis 6 Mol (50 bis 100%iger Überschuß) Phosphoroxychlorid ein.

Das Verfahren wird im einzelnen in der Weise durchgeführt, daß man in vorgelegtes Phosphoroxychlorid unter Kühlung im Temperaturbereich von etwa 0°C bis 20°C Dimethylformamid zutropft. Das 2,4-Thiazoldindion kann entweder vor oder auch erst nach der Dimethylformamid-Zugabe zugefügt werden. Das Reaktionsgemisch wird dann so lange bei Rückflußtemperaturen (ca. 110—120°C) erhitzt, bis die Chlorwasserstoffgas-Entwicklung beendet ist. Die Reaktionsdauer beträgt, je nach den Mengenverhältnissen der

Reaktionsteilnehmer, nach Ansatzgröße usw. zwischen etwa einer und zehn Stunden. Es kann für das Verfahren von Vorteil sein, nach Vereinigung der Reaktionspartner vor dem Aufheizen zunächst einmal eine Zeitlang (z.B. etwa eine Stunde) bei Raumtemperatur oder darunter nachzurühren. Weiterhin kann es vorteilhaft sein, das Erhitzen bis auf Rückflußtemperaturen nicht in einem Zuge durchzuführen, sondern in Phasen stärkerer Chlorwasserstoffgas-Entwicklung, z.B. im Bereich um 80°C, für eine Weile (z.B. etwa eine bis zwei Stunden) zu unterbrechen und erst nach dem Abklingen der Reaktion weiterzuheizen.

Die Aufarbeitung erfolgt in üblicher Weise, indem man vorsichtig auf Eis oder unter Außenkühlung in kaltes Wasser austrägt und den 2,4-Dichlor-5-thiazolcarboxaldehyd mit einem organischen, mit Wasser nicht mischbaren Lösungsmittel (z.B. Methylenchlorid oder Diethylether) extrahiert. Die Reinigung des nach dem Abdampfen des organischen Lösungsmittels verbleibenden rohen 2,4-Dichlor-5-thiazolcarboxaldehyds kann nach allen gebräuchlichen Methoden erfolgen wie Destillation, Kristallisation (z.B. aus Petrolether) oder auf chromatographischem Wege. Die Ausbeuten betragen 50—60% der Theorie an isoliertem, gaschromatographisch reinem Produkt.

2,4-Dichlor-5-thiazolcarboxaldehyd (II) kann als Zwischen-produkt zur Synthese von bekannten herbiziden Wirkstoffen verwendet werden.

Hierzu wird der Aldehyd in üblicher Weise, beispielsweise durch Umsetzung bei 0—50°C mit 1—1,1 Mol Hydroxylamin — zweckmäßig in Form des Hydrochlorids — pro Mol Aldehd, bevorzugt, in Waser als Lösungsmittel, zunächst in sein Oxim der Formel (IV)

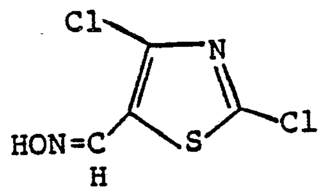

(IV)

umgewandelt. Das Oxim (IV) ist gleichfalls neu und ist ebenfalls Gegenstand der Erfindung. Es kann, ebenfalls nach gebräuchlichen Methoden, z.B. durch Erhitzen mit 1—20 Mol, bevorzugt 1—10 Mol Acetanhydrid pro Mol Oxim bei 100—150°C und 1—5 bar, bevorzugt bei Rückflußtemperatur unter Normaldruck, zum bekannten Nitril der Formel (V)

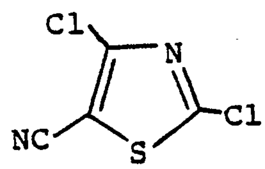

(V)

dehydratisiert werden. Das Acetanhyhdrid dient gleichzeitig als Lösungs- und Verdünnungsmittel. Dieses Nitril kann, wie in der DE—OS 30 38 608 beschrieben, mit Hydroxyessigsäureamiden zu herbiziden Wirkstoffen vom Typ der Thiazolyloxyacetamide, z.B. mit Hydroxyessigsäure-N-methylanilid zu O-(2,4-Dichlor-5-cyano-thiazol-2-yl)-oxyessigsäure-N-methylanilid der Formel (VI)

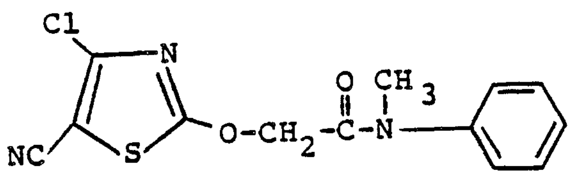

(VI)

umgesetzt werden.

Das neue Verfahren zur Herstellung des Nitrils (V) — auf dem wege (III) → (II) → (IV) → (V) — ist den älteren Herstellungsmethoden (vgl. DE—OS 30 38 806) technisch überlegen, so daß die herbiziden Wirkstoffe des Typs (VI) durch die Erfindung auf wesentlich bessere Weise zugänglich geworden sind.

## Herstellungsbeispiele

A) Herstellung von 2,4-Dichlor-5-thiazolcarboxaldehyd (II):

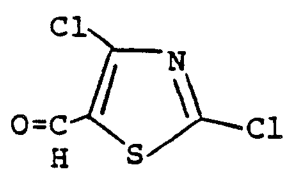

(II)

Beispiel 1

Zu einer Suspension von 46,8 g (0,4 Mol) 2,4-Thiazolidindion (III) in 368 g (2,4 Mol) Phosphoroxychlorid ($POCl_3$) werden bei 10 bis 20°C unter Rühren in 15 Minuten 32,1 g (0,44 Mol) Dimethylformamid getropft. Nach beendeter Zugabe läßt man eine Stunde bei Raumtemperatur nachrühren. Danach heizt man auf 80 bis 90°C auf und rührt eine weitere Stunde bei 80 bis 90°C. Anschließend wird bis auf Rückflußtemperatur (ca. 115°C) erhitzt und dort bis zum Ende der Gasentwicklung weitergerührt (ca. 4 Stunden). Nach dem Abkühlen wird die Reaktionsmischung langsam in 2 kg Eis eingerührt. Man extrahiert dreimal mit je 500 ml Methylenchlorid. Die vereinigten organischen Phasen werden mit wäßriger Natriumhydrogencarbonatlösung gewaschen, über Natriumsulfat getrocknet und durch Abdestillieren des Lösungsmittels im Vakuum eingeengt. Das zurückbleibende braune Öl, das nach einiger Zeit teilweise kristllisiert, wird zunächst vorgereinigt, indem im Wasserstrahlvakuum alle bis zu einer Badtemperatur von etwa 180°C destillierbaren Anteile abdestilliert werden. Das farblose Destillat, das bei Raumtemperatur weitgehend durchkristallisiert, wird zur Entfernung geringer Mengen Flüssiganteile auf Ton getrocknet. Man erhält so 42,8 g (≙ 58,8% der Theorie) gaschromatographisch reinen 2,4-Dichlor-5-thiazolcarboxaldehyd (II). Schmelzpunkt nach Umkristillisation aus Petrolether: 48—49°C.

Beispiel 2

Zu einer Suspension von 250 g (2,14 Mol) 2,4-Thiazolidindion (III) in 1477 g (9,62 Mol) Phosphoroxychlorid werden bei 10 bis 20°C unter Rühren in 15 Minuten 160 g (2,19 Mol) Dimethylformamid getropft. Nach beendeter Zugabe heizt man auf 80 bis 85°C auf und rührt eine Stunde bei 80 bis 85°C. Anschließend wird bis auf Rückflußtemperatur (ca. 115°C) erhitzt und dort bis zum Ende der Gasentwicklung weitergerührt (ca. 8 Stunden). Nach dem Abkühlen wird die Reaktionsmischung langsam in 5 Liter Wasser eingerührt, wobei durch Außenkühlung ein Temperaturbereich von 10 bis 20°C eingehalten wird. Anschließend wird dreimal mit zusammen etwa 5 Liter Methylenchlorid extrahiert. Die vereinigten organischen Phasen werden analog Beispiel 1 aufgearbeitet. Ausbeute an gaschromatographisch reinem 2,4-Dichlor-5-thiazolcarboxaldehyd (II): 216 g (≙ 55,4% der Theorie).

Beispiel 3

Man verfährt zunächst analog Beispiel 2. Nach beendeter Dimethylformamid-Zugabe wird direkt bis auf Rückflußtemperatur erhitzt und dort bis zum Ende der Gasentwicklung weitergerührt (ca. 8 Stunden). Nach dem Abkühlen wird überschüssiges Phosphoroxychlorid im Wasserstrahlvakuum abdestilliert. Das zurückbleibende braune Öl wird mit 2 Liter Methylenchlorid versetzt. Unter intensivem Rühren (Edelstrahlrührer) tropft man nun 2 Liter Wasser zu, vobei durch Außenkühlung die Temperatur des Reaktionsgemisches zwischen 10 und 20°C gehalten wird. Nach Abtrennung der organischen Phase wird die wäßrige Phase noch dreimal mit je 1 Liter Methylenchlorid extrahiert. Die vereinigten organischen Phasen werden analog Beispiel 1 aufgearbeitet. Ausbeute an gaschromatographisch reinem 2,4-Dichlor-5-thiazolcarboxaldehyd (II): 211 g (≙ 54,2% der Theorie).

B) Herstellung von 2,4-Dichlor-5-thiazolcarbonitril (V):

*1. Stufe:* Herstellung von 2,4-Dichlor-5-thiazolcarboxaldehyd-oxim (IV)

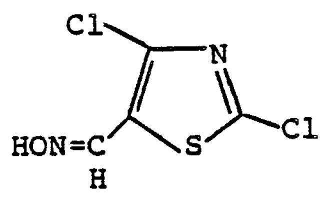

(IV)

Eine Lösung von 185 g (2,2 Mol) Natriumhydrogencarbonat in 5 Liter Wasser wird bei Raumtemperatur unter Rühren zuerst in Anteilen mit 153 g (2,2 Mol) Hydroxylaminhydrochlorid, dann mit einer Lösung von 364 g (2 Mol) 2,4-Dichlor-5-thiazolcarboxaldehyd (II) in 1 Liter Ethanol versetzt. Nach 1 bis 2 Minuten fällt ein voluminöser, farblosser Niederschlag aus. Nach einstündigem Nachrühren wird abgesaugt, mit Wasser gewaschen und getrocknet. Man erhält 390 g (≙ 99% der Theorie) 2,4-Dichlor-5-thiazol-carboxaldehyd-oxim (IV) vom Schmelzpunkt etwa 160°C. Es kann ohne weitere Reinigung zum Nitril dehydratisiert werden.

*2. Stufe:* Herstellung von 2,4-Dichlor-5-thiazolcarbonitril (V):

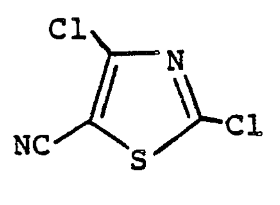

(V)

400 g (2,03 Mol) rohes 2,4-Dichlor-5-thiazolcarboxaldehyd-oxim (IV) werden mit 2 Liter Acetanhydrid 4 Stunden bei Rückflußtemperatur (137°C) gerührt. Fraktionierung an einer 1 m langen, silberverspiegelten Destilationskolonne ergab bei 112°C/20 mbar 271 g (≙ 75,7% der Theorie) 2,4-Dichlor-5-thiazolcarbonitril (V), massen- und IR-spektroskopisch identisch mit dem in der DE—OS 30 38 608 beschriebenen Produkt (das auch als 2,4-Dichlor-5-cyano-thiazol bezeichnet werden kann). Die Verbindung erstarrt bei Raumtemperatur zu farblosen Kristallen, die aus Petrolether umkristallisiert werden können. Schmelzpunkt: 34—35°C.

**Patentansprüche**

1. 2,4-Dichlor-5-thiazolcarboxaldehyd der Formel (II)

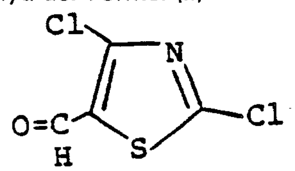

(II)

1. Verfahren zur Herstellung von 2,4-Dichlor-5-thiazolcarboxaldehyd (II), dadurch gekennzeichnet, daß man 2,4-Thiazolidindion der Formel (III)

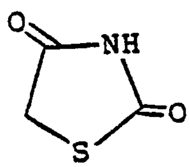

(III)

mit 1—1,5 Mol Dimethylformamid und 3—10 Mol Phosphoroxychlorid bei der Rückflußtemperatur des Reaktionsgemisches (etwa 115°C) bis zum Ende der Chlorwasserstoffgas-Entwicklung umsetzt und anschließend hydrolytisch aufarbeitet.

3. Verfahren nach Anspruch 2, dadurch gekennzeichnet, daß man 1—1,1 Mol Dimethylformamid pro Mol 2,4-Thiazolidindion (III) einsetzt.

4. Verfahren nach Anspruch 2, dadurch gekennzeichnet, daß man 4,5—6 Mol Phosphoroxychlorid pro Mol 2,4-Thiazolidindion (III) einsetzt.

5. 2,4-Dichlor-5-thiazolcarboxaldehyd-oxim der Formel (IV)

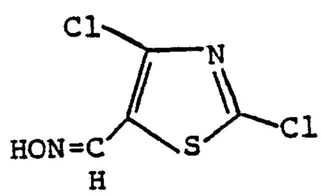

(IV)

6. Verfahren zur Herstellung von 2,4-Dichlor-5-thiazolcarboxaldehyd-oxim (IV) gemäß Anspruch 5, dadurch gekennzeichnet, daß man 2,4-Dichlor-5-thiazolcarboxaldehyd (II) gemäß Anspruch 1 in an sich bekannter Weise mit Hydroxylamin — zweckmäßig in Form des Hydrochlorids — umsetzt.

**Revendications**

1. 2,4-dichloro-5-thiazolecarboxaldéhyde suivant la formule (II)

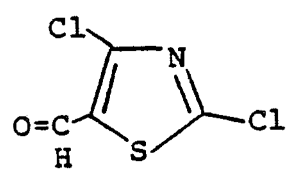

(II)

2. Procédé de préparation du 2,4-dichloro-5-thiazolecarboxaldéhyde (II), caractérisé en ce que l'on fait réagir la 2,4-thiazolidinedione de la formule (III)

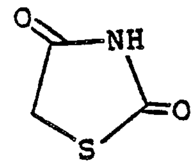

(III)

avec 1 à 1,5 mole de diméthylformamide et 3 à 10 moles d'oxychlorure de phosphore à la température de reflux du mélange réactionnel (environ 115°C) jusqu'à la fin du dégagement de chlorure d'hydrogène gazeux, et qu'on soumet ensuite le produit obtenu à une hydrolyse.

3. Procédé selon la revendication 2, caractérisé en ce que l'on utilise 1 à 1,1 mole de diméthylformamide pour chaque mole de 2,4-thiazolidinedione (III).

4. Procédé selon la revendication 2, caractérisé en ce que l'on utilise 4,5 à 6 moles d'oxychlorure de phosphore pour chaque mole de 2,4-thiazolidinedione (III).

4. 2,4-dichloro-5-thiazolecarboxaldéhyde-oxime de la formule (IV)

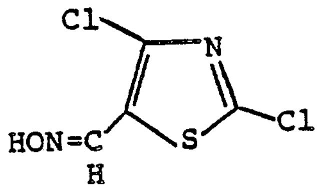

(IV)

6. Procédé de préparation du 2,4-dichloro-5-thiazolecarboxaldéhyde-oxime (IV) selon la revendication 5, caractérisé en ce que l'on fait réagir le 2,4-dichloro-5-thiazolecarboxaldéhyde (II) selon la revendication 1 d'une manière connue en soi, avec de l'hydroxylamine, de préférence, sous forme de chlorhydrate.

## Claims

1. 2,4-Dichloro-5-thiazolecarboxaldehyde of the formula (II)

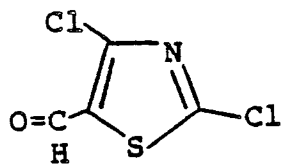

(II)

2. Process for the preparation of 2,4-dichloro-5-thiazolecarboxaldehyde (II), characterised in that 2,4-thiazolidinedione of the formula (III)

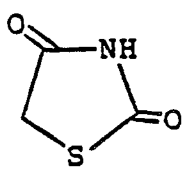

(III)

is reacted with 1—1.5 mol of dimethylformamide and 3—10 mol of phosphorus oxychloride at the reflux temperature of the reaction mixture (about 115°C) until evolution of hydrogen chloride gas has ended, and the mixture is then worked up hydrolytically.

3. Process according to Claim 2, characterised in that 1—1.1 mol of dimethylformamide is employed per mol of 2,4-thiazolidinedione (III).

4. Process according to Claim 2, characterised in that 4.5—6 mol of phosphorus oxychloride are employed per mol of 2,4-thiazolidinedione (III).

5. 2,4-Dichloro-5-thiazolecarboxaldehyde oxime of the formula (IV)

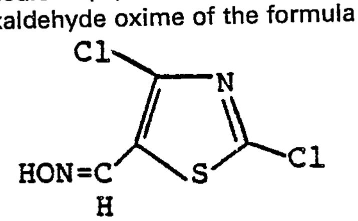

(IV)

6. Process for the preparation of 2,4-dichloro-5-thiazolecarboxaldehyde oxime (IV) according to Claim 5, characterised in that 2,4-dichloro-5-thiazolecarboxaldehyde (II) according to Claim 1 is reacted in a manner known per se with hydroxylamine, advantageously in the form of its hydrochloride.